# EUROPEAN PATENT SPECIFICATION

(11) **EP 4 186 575 B1**
(45) Date of publication and mention of the grant of the patent: **30.10.2024**
(21) Application number: 22020513.2
(22) Date of filing: 25.10.2022
(51) Int. Cl.: B01D 11/02, A61K 36/61

(54) **INTEGRATED PROCESS FOR EXTRACTING BIOACTIVE COMPOUNDS FROM EUCALYPTUS BIOMASS**
INTEGRIERTES VERFAHREN ZUR EXTRAKTION BIOAKTIVER VERBINDUNGEN AUS EUKALYPTUSBIOMASSE
PROCÉDÉ INTÉGRÉ D'EXTRACTION DE COMPOSÉS BIOACTIFS À PARTIR DE BIOMASSE D'EUCALYPTUS

(30) Priority: 30.11.2021 PT 2021117613
(43) Date of publication of application: 31.05.2023
(73) Proprietor: RAIZ - Instituto De Investigação Da Floresta E Papel, 3800-783 Eixo, Aveiro (PT); Universidade de Aveiro, 3810-193 Aveiro (PT); Universidade de Coimbra, 3004-531 Coimbra (PT)
(72) Inventor: De Oliveira Rodrigues Pinto, Paula Cristina, 3800-783 Eixo (PT); Gaspar, Alexandre Miguel Ricardo, 3800-783 Eixo (PT); De Pascoal Neto, Carlos, 3800-783 Eixo (PT); Batista, Maria Teresa Pereira Marques, Coimbra (PT); Pereira, Cláudia Maria Fragão, Coimbra (PT); Reis Moreira, Patrícia Raquel, Coimbra (PT); Da Silva Couto, Lígia Maria Ribeiro Pires Salgueiro, Coimbra (PT); De Teixeira Cruz Rosete, Maria Teresa, Coimbra (PT); Figueirinha, Artur Manuel Bordalo Machado, Coimbra (PT); Santos, Sónia Andreia Oliveira, 3810-193 Aveiro (PT); Silvestre, Armando Jorge Domingues, 3810-193 Aveiro (PT); Da Silva, Artur Manuel Soares, 3810-193 Aveiro (PT); Oliveira, Cátia Sofia Domingues, 3810-193 Aveiro (PT)

(56) References cited:
- WO-A1-2013/160881
- WO-A2-2007/075580
- CN-A- 105 176 688
- US-A1- 2020 164 096
- US-B2- 9 402 407

## Description

### FIELD OF THE INVENTION

The invention relates to the field of production processes of extracts enriched in specific families of bioactive compounds, particularly an integrated process for obtaining said extracts from eucalyptus biomass.

### BACKGROUND OF THE INVENTION

In addition to being an essential raw material in the production of pulp and paper, eucalyptus biomass is also a relevant source of value-added compounds.

Among these value-added compounds there are phenolic compounds, which include flavonoids, phenolic acids, aldehydes, cinnamic acids, coumarins and chromones, benzophenones, xanthones and tannins (Martins et al., Bioactive phenolic compounds: Production and extraction by solid -state fermentation. A review, Biotechnology Advances 29 (2011) 365-373; Sónia Andreia Oliveira Santos, Phenolic compounds from forest industrial by-products, Doctoral Thesis, Universidade de Aveiro, 2012). Phenolic compounds owe their added value to their antimicrobial and antitumor activities, as well as their antioxidant activity. Additionally, its anti-inflammatory, anti-allergic and anti-hemorrhagic effects are reported (Kim et al., Ellagic acid rhamnosides from the stem bark of Eucalyptus globulus, Phytochemistry 2001 Jun;57(4):587-91; Mukhtar et al., Exceptional activity of tannic acid among naturally occurring plant phenols in protecting against 7,12-dimethylbenz(a)anthracene-, benzo(a)pyrene-, 3-methylcholanthrene-, and N-methyl-N-nitrosourea-induced skin tumorigenesis in mice, Cancer Res. 1988 May 1;48(9):2361-5; Hertog et al., Optimization of a quantitative HPLC determination of potentially anticarcinogenic flavonoids in vegetables and fruits, J. Agric. Food Chem. 1992, 40 , 9, 1591-1598).

Because of these properties, phenolic compounds are included in herbal medicines and dietary supplements (Ndhlala et al., Antioxidants: Fascinating or Mythical Biomolecules? Molecules 2010, 15, 6905-6930; Pizzi et al., Tannins: Major Sources, Properties and Applications. In Monomers, Polymers and Composites from Renewable Resources; Belgacem, M.N., Gandini, A., Eds.; Elsevier: Oxford, U.K., 2008; 179-199).

Another type of value-added compounds present in eucalyptus biomass are triterpene compounds, such as betulinic acid, betulinic acid, 3-acetylbetulinic acid, 3-acetylbetulinic acid, ursolic acid, 3-acetylurosolic acid, oleanolic acid, 3-acetyloleanolic acid, β-amyrin, α-amyrin, β-sitosterol, α-amyrenone, lupeol (Domingues et al., High value triterpenes compounds from the outer barks of several Eucalyptus species cultivated in Brazil and in Portugal, Industrial Crops and Products 33, 2011, 158-164; Domingues et al., Eucalyptus biomass residues from agro-forest and pulping industries as sources of high-value triterpenes compounds, Cellulose Chem. Technol., 45, 2011, 475-481.), which, in turn, have antioxidant, anti-microbial, anti-viral, anti-allergic, anti-pruritic and anti-angiogenic properties (F. Alihosseini, Plant-based compounds for antimicrobial textiles, Antimicrobial Textiles, Woodhead Publishing Series in Textiles, 2016, Pages 155-195).

Another product from eucalyptus biomass is its essential oil, with a typical composition that includes monoterpenes such as 1,8-cineole (eucalyptol), α-Pinene, β-Pinene, limonene, camphene, p-cymene, and sequiterpenes. such as aromadendrene or globulol (Luís et al., Chemical composition, antioxidant, antibacterial and anti-quorumsensing activities of E. globulus and E. radiata essential oils, Industrial Crops and Products 79 (2016) 274-282), with biological activities proven, for example, anti-microbial, anti-viral, fungicidal, insecticidal, anti-inflammatory, anti-nociceptive, antioxidant and phytotoxic (Zhang et al., Chemistry and bioactivity of Eucalyptus essential oils, Allelopathy Journal 25 (2) : 313-330 (2010)).

The beneficial characteristics of these families of compounds have led to the development of methods that allow their extraction, either from eucalyptus biomass or from other raw materials of plant origin.

One of the most used methods for phenolic and triterpene compounds is solid-liquid extraction, which consists of bringing the biomass into contact with a solvent such as, for example, water or polar and non-polar solvents or different mixtures thereof. The extraction process can take place at different temperatures, solid/liquid ratios and at different extraction times (Ballard et al., Optimizing the Extraction of Phenolic Antioxidants from Peanut Skins Using Response Surface Methodology, J. Agric. Food Chem. 2009, 57, 8, 3064-3072).

Another common extraction process is the Soxhlet extraction, in which a solvent, or mixtures thereof, is continuously recycled and brought into contact with the biomass, through consecutive cycles of boiling and condensation (Alara et al. Soxhlet extraction of phenolic compounds from Vernonia cinerea leaves and its antioxidant activity, Journal of Applied Research on Medicinal and Aromatic Plants, 11, 2018, 12-17).

These two extraction techniques are characterized by requiring a high consumption of solvents or their mixtures, and by long extraction times (Mussatto et al., Chapter 11 - Generating Biomedical Polyphenolic Compounds from Spent Coffee or Silverskin, Coffee in Health and Disease Prevention 2015, Pages 93-106).

There is also the possibility of extraction using supercritical fluids. This technique is faster, more selective for less polar (or non-polar) compounds and, additionally, with a lower environmental impact (Sánchez-Camargo, Supercritical Fluid Extraction, Reference Module in Chemistry, Molecular Sciences and Chemical Engineering 2014). However, in general, supercritical extraction requires the use of expensive equipment and its configurations and operations are considered complex (Al Jitan et al., Chapter 13 - Phenolic Acids From Plants: Extraction and Application to Human Health, in Studies in Natural Products Chemistry, Volume 58, 2018, Pages 389-417) .

In turn, hydrodistillation is used for the extraction of essential oils, consisting of the immersion of the raw material in water and exposure to heat up to the boiling point of the mixture, causing the release of the essential oil from the raw material. The compounds are then separated by density difference after condensation of the mixture (kharraf et al., Two Extraction Methods of Essential Oils: Conventional and Non-conventional Hydrodistillation, Journal of Essential Oil Bearing Plants, 23 (5) 2020, 870 - 889).

A brief search makes it possible to find different works that describe obtaining extracts rich in value-added compounds from plant biomass, using the aforementioned extraction techniques and solvents.

Patent application EP1687326A2 describes a method for obtaining triterpenoids, such as lupeol and betulinic acid, from plant biomass such as birch bark, which involves the use of at least two organic solvents in extraction processes. Solvents considered in the invention described in this patent application include aromatic hydrocarbons such as xylene, o-xylene, m-xylene, p-xylene, toluene, benzene and combinations thereof, chlorinated solvents such as chloroform and dichloromethane, as well as other organic solvents compounds such as ethyl t-butyl ether and ethyl acetate.

Patent JP5602346B2 describes a method of obtaining a eucalyptus extract, more particularly a fraction rich in mono and sesquiterpene compounds abundant in the essential oil, through several extraction steps involving organic solvents chosen from n-hexane, toluene, dichloroethane, chloroform, ethyl acetate, acetone, methanol, ethanol, propanol, butanol, ethylene glycol, propylene, glycol, butylene glycol and the like.

Patent application EP1960329A2 describes a process for obtaining different compounds from vegetable matter, such as essential oil, phenolic compounds, sugar derivatives, triterpenes compounds and organic acids. The described process involves the application of different extraction steps (two, three or more) with the same type of solvent, or with different choices or mixtures thereof. It is cited, for example, the use of ethyl acetate, sodium hydroxide and hexane solutions.

Patent application WO2013160881A1 describes a method for obtaining an extract rich in triterpenic acids from eucalyptus barks, involving the solid-liquid extraction of the bark with hexane and the fractionation of the crude extract by means of an alkaline solution, followed by separation of the aqueous phase, its filtration, acidification and finally isolation of the enriched triterpenic acids.

Patent application CN105176688A discloses a method for extracting eucalyptus oil which involves drying the eucalyptus leaves in the shade, treating them with cellulase, and then distilling and extracting the oil.

US20200164096A1 depicts an air freshener composition that uses a combination of specific plant extracts to effectively clean and freshen the air, where the extract is produced by crushing leaves and, adding ethanol and then extracting the resultant mixture using microwave heating and filtering and concentrating the resultant filtrate under reduced pressure.

Academic works report the use of carbon dioxide extractions under supercritical conditions, as is the case of the study by Domingues et al. (Supercritical Fluid Extraction of Eucalyptus globulus Bark-A Promising Approach for Triterpenoid Production, Int. J. Mol. Sci. 2012, 13(6), 7648-7662) or de Santos et al. (Santos et al., Supercritical fluid extraction of phenolic compounds from Eucalyptus globulus Labill bark, The Journal of Supercritical Fluids, 2012, 71-79) for the extraction of, respectively, triterpene compounds and phenolic compounds from E. globulus bark. In turn, Ferreira and co-authors (Phenolic Compounds in Extracts from Eucalyptus globulus Leaves and Calendula officinalis Flowers, Journal of Natural Products and Resources 2(1) (2016) 53-57), describe the extraction of E. globulus leaves with chosen solvents such as chloroform, ethanol and methanol.

Thus, there is a predominance of extraction processes for value-added compounds that use organic solvents and often their mixtures, in several extraction steps. As is well known, organic solvents are toxic to humans, animals and the environment, and there is a growing demand for less adverse alternatives. The existing extraction processes limit the type of extract obtained, and the processes developed are mostly focused on the production of extracts enriched in a certain type of compounds.

It is therefore extremely important to develop an extraction method that allows obtaining different extracts, enriched in certain families of value-added bioactive compounds, with different polarities and different bioactive properties, from the same biomass, maximizing the generation of added value, and which allows for a reduction in the use of organic solvents, both in quantity and in their number and, consequently, also leading to a decrease in the costs involved in the process, with regard to the recovery of organic solvents and reduction of environmental impacts. Moderate temperature and pressure conditions are also desirable. These requirements are satisfied by the process described in this patent application.

### SUMMARY OF THE INVENTION

The present invention is directed to an integrated process for extracting bioactive compounds from eucalyptus biomass, comprising the following steps:
a) selection of leaves of eucalyptus;
b) hydrodistillation of the leaves of eucalyptus for the production of an extract with phenolic compounds obtained from the residual water of hydrodistillation, an essential oil of eucalyptus and a hydrodistilled biomass;
c) separating the extract with phenolic compounds obtained from the residual water of hydrodistillation, the essential oil of eucalyptus and the hydrodistilled biomass obtained in step b);
d) extracting the biomass resulting from step c) and/or eucalyptus bark with the eucalyptus essential oil resulting from step c) until obtaining an extract of triterpene compounds dissolved in eucalyptus essential oil and an extracted biomass;
e) hydrodistillation of the extracted biomass resulting from step d) until obtaining an extract with phenolic compounds obtained from the residual water of the hydrodistillation, the eucalyptus essential oil and a hydro-distilled biomass;
f) extraction of the biomass resulting from step e) with water and/or with an aliphatic alcohol and/or with mixtures of water and aliphatic alcohols until obtaining an extract of phenolic compounds and a post-extraction biomass.

### BRIEF DESCRIPTION OF THE DRAWINGS

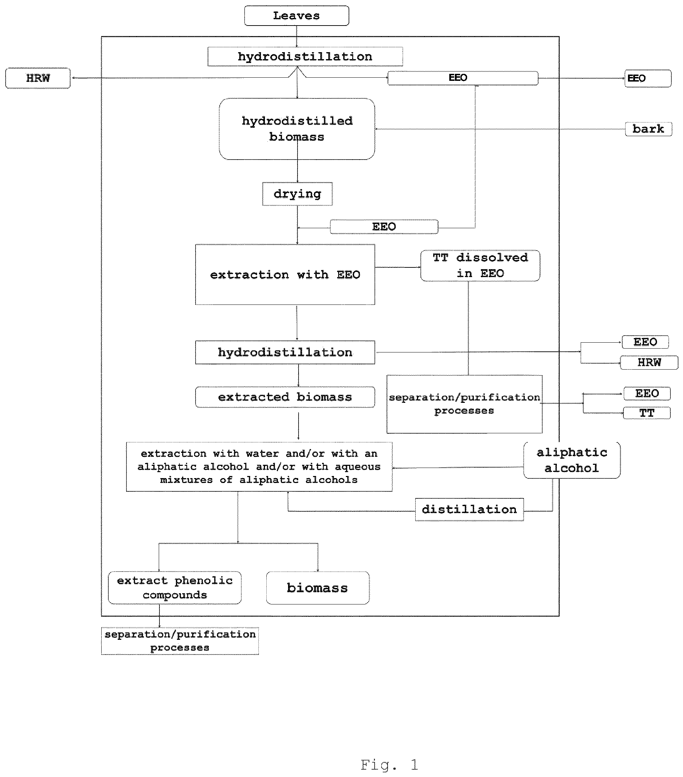

Figure 1. Diagram of the integrated process for simultaneous production of value-added bioactive compounds from eucalyptus biomass as described in this invention. HRW - extract with phenolic compounds obtained from residual water from hydrodistillation; EB - eucalyptus biomass; EEO - eucalyptus essential oil; TT - extract of triterpene compounds.

### DETAILED DESCRIPTION OF THE INVENTION

The invention described in this document concerns an integrated process for the simultaneous production of value-added bioactive compounds from the same eucalyptus biomass, as described in this patent application and claim 1, which allows obtaining a variety of extracts, enriched in specific families of compounds and with different degrees of polarity, using a smaller number and respective amount of organic solvents. The problems of solvent recovery and of possible toxicity and/or environmental contamination are thus minimized. It also allows a reduction in the costs associated with the production of extracts rich in value-added bioactive compounds. The process described in this document involves an integrated process that includes extraction stages, which enhance the production of different extracts, enriched in different families of value-added compounds from the same biomass, and the recirculation of one of the products of this extraction process, reducing the problems associated with the recovery of organic solvents and the associated costs.

In fact, and as detailed below, in the process described here, the base biomass, for example eucalyptus leaves or bark, generates itself the solvent that will then allow the extraction of the triterpene compounds, that is, the essential oil, obtained by hydrodistillation of the original biomass and its subsequent use as a solvent in the sequential extraction of triterpene compounds.

In a preferred embodiment of the invention, the process further comprises a step of drying the hydrodistilled biomass resulting from step c).

In a preferred embodiment of the invention, the extraction of step f) is carried out in a medium acidified by an organic acid.

In a preferred embodiment of the invention, the extraction of step f) is carried out with aliphatic alcohols selected from the group consisting of methanol, ethanol and propanol.

In a preferred embodiment of the invention, the process further comprises a step of purifying the extract of triterpene compounds dissolved in eucalyptus essential oil resulting from step d) until obtaining eucalyptus essential oil and an extract of triterpene compounds.

In a preferred embodiment of the invention, the step of purifying the extract of triterpene compounds dissolved in eucalyptus essential oil is carried out through purification methods, individually or sequentially, selected from the group consisting of filtration, alkaline extraction, acidification and precipitation.

In a preferred embodiment of the invention, the process further comprises a step of purifying the extract of phenolic compounds obtained in step f).

In a preferred embodiment of the invention, the step of purifying the extract of phenolic compounds is carried out through purification methods, individually or sequentially, selected from the group consisting of filtration, evaporation and lyophilization.

In a preferred embodiment of the invention, the process further comprises collecting the eucalyptus essential oil obtained in steps c) and e) for subsequent rectification and/or concentration and/or purification.

In a preferred embodiment of the invention, the process further comprises collecting the extracts with phenolic compounds obtained from the residual water of the hydrodistillation obtained in steps c) and e) for subsequent rectification and/or concentration and/or purification.

Within the scope of the present invention, a bioactive compound refers to compounds present in plant materials with beneficial effects on human health, such as antioxidant, antimicrobial, anti-tumor, anti-inflammatory and anti-allergic effects, among other beneficial effects.

Within the scope of the present invention, extract with phenolic compounds obtained from the residual water of hydrodistillation refers to a non-volatile extract obtained from the residual water resulting from the hydrodistillation process of leaves to collect the essential oil, which has phenolic compounds in its constitution, such as, for example and not limited to flavonoids, phenolic acids, aldehydes, cinnamic acids, coumarins and chromones, benzophenones, xanthones and tannins.

Within the scope of the present invention, essential oil refers to the product obtained after subjecting plant biomass to the hydrodistillation process, where water is evaporated simultaneously with the essential oil and after condensation they are separated into two phases, one of which is the essential oil. This consists of a complex mixture of biologically active compounds.

Selected eucalyptus biomass, leaves, is subjected to hydrodistillation until the production of an extract with phenolic compounds obtained from the residual water of hydrodistillation and an essential eucalyptus oil. The hydro-distilled biomass, that is, after the hydrodistillation process, is dried before its subsequent extraction. Eucalyptus essential oil obtained in the first hydrodistillation is used to extract the leaves resulting from the drying step and/or bark. This extraction results in an extract of triterpene compounds dissolved in eucalyptus essential oil and extracted biomass. This biomass, be it leaves and/or bark, is again hydrodistilled to recover the essential oil that was retained in the biomass of the previous step, obtaining hydrodistilled biomass, eucalyptus essential oil and an extract with phenolic compounds obtained from the residual water of the hydrodistillation.

The resulting biomass is sent to another extraction with water and/or with aliphatic alcohols and/or with mixtures of water and aliphatic alcohols. This last extraction, in addition to the extract rich in triterpene compounds and the constituents of eucalyptus essential oil obtained during the process steps described above, allows for an extract of phenolic compounds to be obtained.

The extract of triterpene compounds dissolved in eucalyptus essential oil and resulting from extraction with the essential oil is subjected to one or more purification processes. The extract of phenolic compounds obtained in the final step is subjected to one or more purification processes.

The different streams of the product ranges obtained in this integrated process, whether from eucalyptus essential oil or from extract with phenolic compounds obtained from the residual water of hydrodistillation, are collected individually and subjected, according to their constitution, to rectification, concentration and/or purification processes.

Different purification processes can be selected, individually or sequentially, among, for example, but not limited to, filtration, alkaline extraction, acidification and precipitation.

The process described in this patent application thus allows the production of a variety of value-added compounds from the same biomass, enabling the recirculation of different by-products and solvents, thus minimizing production costs and subsequent environmental impacts. In fact, one of the products obtained, the essential oil, is used for the extraction, in this integrated process, of another family of products. Triterpene compounds, eucalyptus essential oil, extracts with phenolic compounds obtained from the residual water from hydrodistillation and phenolic compounds are obtained through the sequential and integrated steps of the process in question.

### Example

### Extraction of the fraction rich in triterpene compounds with the essential oil

The biomass extracted by hydrodistillation was lyophilized, crushed, and then extracted with the oil, such as eucalyptus essential oil and/or 1,8-cineole. Approximately 7 g of eucalyptus leaves and/or bark were extracted with 25 mL of eucalyptus essential oil and/or 1,8-cineole, at room temperature, protected from light and with stirring for up to 24 hours.

Biomass and the liquid fraction (extract enriched in triterpene compounds dissolved in essential oil) were separated by pressing separation process, obtaining an extract rich in triterpene compounds dissolved in essential oil and/or 1,8-cineole. The total triterpenes content was 28.4 g kg⁻¹ of dry biomass, containing 18.3 g kg⁻¹ of ursolic acid dry biomass, 6.0 g kg⁻¹ of oleanolic acid dry biomass, 2.6 g kg⁻¹ of dry biomass of betulinic acid and 1.4 kg⁻¹ of betulinic acid biomass.

### Fractionation of the crude extract of essential oil from biomass

A volume of 15 mL of crude essential oil extract was extracted with 15 mL of 0.1 M aqueous sodium hydroxide (NaOH), this L-L extraction being repeated between 9 and 16 times. The aqueous phase was separated from the organic phase (essential oil and/or cineole) by decanting. It was further centrifuged, and then vacuum filtered through a Whatman 6 cellulose filter (3 pm), resulting in a clearer solution. Said solution was acidified to pH < 3 with 2M sulfuric acid (H₂SO₄), resulting in a precipitated suspension. The precipitate was vacuum filtered, washed with 1 L of distilled water until neutral pH, and dried at 105 °C for 3 h.

292 mg of extract were obtained, consisting of 65.7% ursolic acid, 18.1% oleanolic acid, 6.1° betulinic acid and 2.3% betulinic acid.

### Extraction of phenolic compounds using water, and/or aliphatic alcohols, and/or mixtures of water and aliphatic alcohols

The extracted biomass was sequentially subjected to extraction with water, and/or short chain aliphatic alcohols, and/or mixtures of water and aliphatic alcohols with or without acidification (e.g. with acetic acid), using solvent/biomass ratios, by mass /volume, up to 1:100, with an extraction time of up to 24h and temperatures at least equal to room temperature and up to the boiling temperature of the solvent used.

The solid fraction and the extract rich in phenolic compounds dissolved in the used solvent were separated by filtration, alkaline extraction, acidification and precipitation, and the solid extract obtained by removing the solvent by evaporation, lyophilization and a combination of both.

Overall yields of at least 8.0 % and 11.5 % were observed for extractions with methanol:water and ethanol:water mixtures, respectively, and 12.1 % and 15.0 % for extractions with ethanol and methanol, respectively in terms of kg extract produced per kg of biomass.

**Table 1. Extraction yields with different solvents considered.**

| Solvent | Yield (%) |
|---|---|
| Ethanol | 12,1 |
| Methanol | 15, 0 |
| Ethanol:water | 11,5 |
| Methanol:water | 8,0 |

## Claims

1. An integrated process for extracting bioactive compounds from eucalyptus biomass, comprising the following steps:
a) selection of leaves of eucalyptus;
b) hydrodistillation of the leaves of eucalyptus for the production of an extract with phenolic compounds obtained from the residual water of hydrodistillation, an essential oil of eucalyptus and a hydrodistilled biomass;
c) separating the extract with phenolic compounds obtained from the residual water of hydrodistillation, the essential oil of eucalyptus and the hydrodistilled biomass obtained in step b) ;
d) extracting the biomass resulting from step c) and/or eucalyptus bark with the eucalyptus essential oil resulting from step c) until obtaining an extract of triterpene compounds dissolved in eucalyptus essential oil and an extracted biomass;
e) hydrodistillation of the extracted biomass resulting from step d) until obtaining an extract with phenolic compounds obtained from the residual water of the hydrodistillation, the eucalyptus essential oil and a hydrodistilled biomass;
f) extraction of the biomass resulting from step e) with water and/or with an aliphatic alcohol and/or with mixtures of water and aliphatic alcohols until obtaining an extract of phenolic compounds and a post-extraction biomass.

2. The process according to the preceding claim, wherein it further comprises a step of drying the hydrodistilled biomass resulting from step c) .

3. The process according to any one of the preceding claims, wherein in the extraction of step f) is carried out in a medium acidified by an organic acid.

4. The process according to any one of the preceding claims, wherein in the extraction of step f) is carried out with aliphatic alcohols selected from the group consisting of methanol, ethanol and propanol.

5. The process according to any one of the preceding claims, wherein in it further comprises a step of purifying the extract of triterpene compounds dissolved in eucalyptus essential oil resulting from step d) until obtaining eucalyptus essential oil and an extract of triterpene compounds.

6. The process according to the preceding claim, wherein in the step of purifying the extract of triterpene compounds dissolved in eucalyptus essential oil is carried out through purification methods, individually or sequentially, selected from the group consisting of filtration, alkaline extraction, acidification and precipitation.

7. The process according to any one of the preceding claims, wherein in it further comprises a step of purifying the extract of phenolic compounds obtained in step f).

8. The process according to the preceding claim, wherein the step of purifying the extract of phenolic compounds is carried out through purification methods, individually or sequentially, selected from the group consisting of filtration, evaporation and lyophilization.

9. The process according to any one of the preceding claims, wherein it further comprises collecting the eucalyptus essential oil obtained in steps c) and e) for subsequent rectification and/or concentration and/or purification.

10. The process according to any one of the preceding claims, wherein it further comprises collecting the extracts with phenolic compounds obtained from the residual water of the hydrodistillation obtained in steps c) and e) for subsequent rectification and/or concentration and/or purification.

## Patentansprüche

1. Ein integriertes Verfahren zur Extraktion bioaktiver Verbindungen aus Eukalyptus-Biomasse, das die folgenden Schritte umfasst:
a) Auswahl von Eukalyptusblättern;
b) Hydrodestillation der Eukalyptusblätter zur Herstellung eines Extrakts mit phenolischen Verbindungen, der aus dem Restwasser der Hydrodestillation erhalten wird, eines ätherischen Eukalyptusöls und einer hydrodestillierten Biomasse;
c) Trennen des Extrakts mit phenolischen Verbindungen, der aus dem Restwasser der Hydrodestillation, dem ätherischen Eukalyptusöl und der in Schritt b) erhaltenen hydrodestillierten Biomasse gewonnen wurde;
d) Extraktion der in Schritt c) erhaltenen Biomasse und/oder der Eukalyptusrinde mit dem in Schritt c) erhaltenen ätherischen Eukalyptusöl, bis man einen Extrakt aus in ätherischem Eukalyptusöl gelösten Triterpenverbindungen und eine extrahierte Biomasse erhält;
e) Hydrodestillation der extrahierten Biomasse aus Schritt d) bis zum Erhalt eines Extrakts mit phenolischen Verbindungen aus dem Restwasser der Hydrodestillation, dem ätherischen Eukalyptusöl und einer hydrodestillierten Biomasse;
f) Extraktion der aus Schritt e) resultierenden Biomasse mit Wasser und/oder mit einem aliphatischen Alkohol und/oder mit Mischungen aus Wasser und aliphatischen Alkoholen bis zum Erhalt eines Extrakts mit phenolischen Verbindungen und einer nachextrahierten Biomasse.

2. Das Verfahren nach dem vorhergehenden Anspruch, wobei es ferner einen Schritt des Trocknens der aus Schritt c) resultierenden hydrodestillierten Biomasse umfasst.

3. Verfahren nach einem der vorangehenden Ansprüche, wobei die Extraktion in Schritt f) in einem mit einer organischen Säure angesäuerten Medium durchgeführt wird.

4. Verfahren nach einem der vorhergehenden Ansprüche, wobei die Extraktion von Schritt f) mit aliphatischen Alkoholen, ausgewählt aus der Gruppe bestehend aus Methanol, Ethanol und Propanol, durchgeführt wird.

5. Verfahren nach einem der vorhergehenden Ansprüche, **dadurch gekennzeichnet, dass** es ferner einen Schritt der Reinigung des Extrakts aus Triterpenverbindungen, gelöst in ätherischem Eukalyptusöl aus Schritt d), bis zum Erhalt von ätherischem Eukalyptusöl und einem Extrakt aus Triterpenverbindungen umfasst.

6. Verfahren nach dem vorhergehenden Anspruch, wobei der Schritt der Reinigung des Extrakts aus Triterpenverbindungen, die in dem ätherischen Eukalyptusöl gelöst sind, durch Reinigungsmethoden, einzeln oder nacheinander, durchgeführt wird, die aus der Gruppe ausgewählt sind, die aus Filtration, alkalischer Extraktion, Ansäuerung und Ausfällung besteht.

7. Verfahren nach einem der vorhergehenden Ansprüche, **dadurch gekennzeichnet, dass** es ferner einen Schritt der Reinigung des in Schritt f) erhaltenen Extrakts aus phenolischen Verbindungen umfasst.

8. Verfahren nach dem vorhergehenden Anspruch, wobei der Schritt der Reinigung des Extrakts aus phenolischen Verbindungen durch Reinigungsmethoden, einzeln oder nacheinander, durchgeführt wird, die aus der Gruppe ausgewählt sind, die aus Filtration, Verdampfung und Lyophilisierung besteht.

9. Verfahren nach einem der vorhergehenden Ansprüche, wobei es ferner das Sammeln des in den Schritten c) und e) erhaltenen ätherischen Eukalyptusöls zur anschließenden Rektifikation und/oder Konzentration und/oder Reinigung umfasst.

10. Verfahren nach einem der vorhergehenden Ansprüche, wobei es ferner das Sammeln der Extrakte mit phenolischen Verbindungen, die aus dem Restwasser der in den Schritten c) und e) erhaltenen Hydrodestillation erhalten wurden, für eine anschließende Rektifikation und/oder Konzentration und/oder Reinigung umfasst.

## Revendications

1. Procédé intégré d'extraction de composés bioactifs à partir de la biomasse d'eucalyptus, comprenant les étapes suivantes :
a) sélection des feuilles d'eucalyptus ;
b) hydrodistillation des feuilles d'eucalyptus pour la production d'un extrait avec des composés phénoliques obtenus à partir de l'eau résiduelle de l'hydrodistillation, d'une huile essentielle d'eucalyptus et d'une biomasse hydrodistillée ;
c) séparer l'extrait de composés phénoliques obtenu à partir de l'eau résiduelle de l'hydrodistillation, de l'huile essentielle d'eucalyptus et de la biomasse hydrodistillée obtenue à l'étape b) ;
d) extraction de la biomasse résultant de l'étape c) et/ou de l'écorce d'eucalyptus avec l'huile essentielle d'eucalyptus résultant de l'étape c) jusqu'à l'obtention d'un extrait de composés triterpéniques dissous dans l'huile essentielle d'eucalyptus et d'une biomasse extraite ;
e) hydrodistillation de la biomasse extraite à l'issue de l'étape d) jusqu'à l'obtention d'un extrait de composés phénoliques obtenu à partir de l'eau résiduelle de l'hydrodistillation, de l'huile essentielle d'eucalyptus et d'une biomasse hydrodistillée ;
f) extraction de la biomasse résultant de l'étape e) avec de l'eau et/ou avec un alcool aliphatique et/ou avec des mélanges d'eau et d'alcools aliphatiques jusqu'à l'obtention d'un extrait de composés phénoliques et d'une biomasse post-extraction.

2. Le procédé selon la revendication précédente, dans lequel il comprend en outre une étape de séchage de la biomasse hydrodistillée résultant de l'étape c).

3. Procédé selon l'une quelconque des revendications précédentes, dans lequel l'extraction de l'étape f) est effectuée dans un milieu acidifié par un acide organique.

4. Procédé selon l'une quelconque des revendications précédentes, dans lequel l'extraction de l'étape f) est effectuée avec des alcools aliphatiques choisis dans le groupe constitué par le méthanol, l'éthanol et le propanol.

5. Le procédé selon l'une quelconque des revendications précédentes, dans lequel il comprend en outre une étape de purification de l'extrait de composés triterpéniques dissous dans l'huile essentielle d'eucalyptus résultant de l'étape d) jusqu'à l'obtention d'une huile essentielle d'eucalyptus et d'un extrait de composés triterpéniques.

6. Procédé selon la revendication précédente, dans lequel l'étape de purification de l'extrait de composés triterpéniques dissous dans l'huile essentielle d'eucalyptus est effectuée par des méthodes de purification, individuellement ou séquentiellement, choisies dans le groupe constitué par la filtration, l'extraction alcaline, l'acidification et la précipitation.

7. Procédé selon l'une quelconque des revendications précédentes, dans lequel il comprend en outre une étape de purification de l'extrait de composés phénoliques obtenu à l'étape f).

8. Procédé selon la revendication précédente, dans lequel l'étape de purification de l'extrait de composés phénoliques est effectuée par des méthodes de purification, individuellement ou séquentiellement, choisies dans le groupe constitué par la filtration, l'évaporation et la lyophilisation.

9. Procédé selon l'une quelconque des revendications précédentes, dans lequel il comprend en outre la collecte de l'huile essentielle d'eucalyptus obtenue aux étapes c) et e) pour une rectification et/ou une concentration et/ou une purification ultérieures.

10. Procédé selon l'une quelconque des revendications précédentes, dans lequel il comprend en outre la collecte des extraits avec des composés phénoliques obtenus à partir de l'eau résiduelle de l'hydrodistillation obtenue aux étapes c) et e) pour une rectification et/ou une concentration et/ou une purification ultérieures.
